Europäisches Patentamt

European Patent Office

Office européen des brevets

(11) Veröffentlichungsnummer: **0 399 157**
**A2**

## (12) EUROPÄISCHE PATENTANMELDUNG

(21) Anmeldenummer: **90104687.0**

(22) Anmeldetag: **13.03.90**

(51) Int. Cl.5: **A61K 7/06**

(30) Priorität: **24.05.89 DE 3916917**

(43) Veröffentlichungstag der Anmeldung:
**28.11.90 Patentblatt 90/48**

(84) Benannte Vertragsstaaten:
**DE ES FR GB IT**

(71) Anmelder: **Wella Aktiengesellschaft**
**Berliner Allee 65**
**D-6100 Darmstadt(DE)**

(72) Erfinder: **Gross, Paul**
**Forstweg 54**
**D-6100 Darmstadt(DE)**
Erfinder: **Henze, Hildegard**
**Georgenstrasse 11**
**D-6100 Darmstadt(DE)**

(54) **Schaumförmiges Haarbehandlungsmittel und Verfahren zu seiner Herstellung.**

(57) Gegenstand der Erfindung ist ein schaumförmiges Haarbehandlungsmittel, welches vor der Anwendung durch Vermischen zweier Komponenten hergestellt wird, wobei die erste Komponente in fester Form vorliegt und eine physiologisch verträgliche, wasserlösliche Säure oder ein physiologisch verträgliches, wasserlösliches saures Salz sowie ein physiologisch verträgliches, Carbonat oder Hydrogencarbonat enthält und die zweite Komponente ein wasserhaltiges Haarbehandlungsmittel ist und 0,1 bis 50 Gewichtsprozent einer oberflächenaktiven Substanz enthält.

Weiterhin betrifft die Erfindung ein Verfahren zur Herstellung des schaumförmigen Haarbehandlungsmittels.

EP 0 399 157 A2

## Schaumförmiges Haarbehandlungsmittel und Verfahren zu seiner Herstellung

Die vorliegende Erfindung betrifft ein aus zwei Komponenten erhältliches schaumförmiges Haarbehandlungsmittel sowie ein Verfahren zur Herstellung dieses Mittels.

Aufgrund zahlreicher anwendungstechnischer Vorteile werden schaumförmige Haarbehandlungsmittel in zunehmendem Maße anstelle der bisher üblichen flüssigen oder angedickten Mittel eingesetzt. Derartige schaumförmige Mittel besitzen im Vergleich zu üblichen Mitteln einerseits eine leichtere Dosierbarkeit und können andererseits infolge ihres größeren Volumens gleichmäßiger im Haar verteilt werden, wodurch eine sehr gleichmäßige Wirkung erzielt wird.

Die vorstehend genannten schaumförmigen Mittel werden erhalten, indem man des entsprechende Haarbehandlungsmittel mit einem geeigneten, unter Druck verflüssigten Treibmittel in eine Druckverpackung abfüllt, aus der anschließend das schaumförmige Mittel in der gewünschten Menge entnommen wird. Als Treibmittel werden vorwiegend Fluorchlorkohlenwasserstoffe und leichtflüchtige Alkane verwendet.

Die Verwendung derartiger in Druckverpackungen abgefüllter Mittel (sogenannter Aerosolschaumpräparate) ist jedoch sowohl aus ökologischer Sicht als auch in sicherheitstechnischer Hinsicht problematisch. So gelten beispielsweise Fluorchlorkohlenwasserstoffe als umweltgefährdend, weshalb ihre Verwendung in zunehmendem Maße be schränkt oder auch völlig verboten wird. Leichtflüchtige Kohlenwasserstoffe hingegen besitzen den Nachteil, daß wegen ihrer hohen Feuergefährlichkeit bei der Abfüllung, dem Transport sowie der Lagerung der entsprechenden Mittel besondere Sicherheitsmaßnahmen erforderlich sind. Zudem ist die Herstellung von Aerosolschaumpräparaten wegen des für die Druckverpackungen sowie die Abfüllanlagen erforderlichen großen technischen und sicherheitstechnischen Aufwandes mit hohen Kosten verbunden.

Aus der Literatur, beispielsweise der JP-OS 61-37 718, sind pulverförmige Haarbehandlungsmittel bekannt, die eine organische Säure und ein Hydrogencarbonat oder Carbonat enthalten und sich in Wasser unter Sprudeln auflösen. Ein stabiler, feinporiger Schaum ist mit diesen Mitteln jedoch nicht erzielbar.

Aufgabe der vorliegenden Erfindung ist es daher, ein schaumförmiges Haarbehandlungsmittel zur Verfügung zu stellen, bei dem auch ohne die Verwendung von Druckgaspackungen und Treibgasen ein stabiler, voluminöser und feinporiger Schaum erhalten wird.

Es wurde nunmehr gefunden, daß ein schaumförmiges Haarbehandlungsmittel mit einem stabilen, voluminösen und feinporigen Schaum erhalten wird, wenn vor der Anwendung zu einem wasserhaltigen Haarbehandlungsmittel eine pulverförmige Mischung aus einer physiologisch verträglichen, wasserlöslichen Säure oder eines physiologisch verträglichen, wasserlöslichen sauren Salzes und einem physiologisch verträglichen Carbonat oder Hydrogencarbonat gegeben wird.

Gegenstand der vorliegenden Erfindung ist daher ein schaumförmiges Haarbehandlungsmittel, welches vor der Anwendung durch Vermischen zweier Komponenten hergestellt wird und dadurch gekennzeichnet ist, daß

(1) die erste Komponente in fester Form, als Pulver oder Granulat, vorliegt und eine physiologisch veträgliche, wasserlösliche Säure oder ein physiologisch verträgliches, wasserlösliches saures Salz sowie ein physiologisch verträgliches Carbonat oder Hydrogencarbonat enthält und

(2) die zweite Komponente ein wasserhaltiges Haarbehandlungsmittel ist und 0.1 bis 50 Gewichtsprozent einer oberflächenaktiven Substanz enthält.

Die erste Komponente des erfindungsgemäßen Haarbehandlungsmittels ist zur Verbesserung der Lagerfähigkeit vorzugsweise wasserfrei und liegt vorzugsweise in Form eines feinkörnigen Pulvers vor. Die erste Komponente besteht im wesentlichen aus einer Mischung einer physiologisch verträglichen, wasserlöslichen Säure oder eines physiologisch verträglichen, wasserlöslichen sauren Salzes mit einem physiologisch verträglichen Carbonat oder Hydrogencarbonat.

Als physiologisch verträgliche, wasserlösliche Säure können beispielsweise Zitronensäure, Äpfelsäure, Weinsäure, Ascorbinsäure, Mandelsäure, Glyoxylsäure oder Malonsäure verwendet werden, während als physiologisch verträgliches, wasserlösliches saures Salz zum Beispiel Natriumhydrogentartrat oder Natriumhydrogensulfat in Betracht kommen.

Geeignete physiologisch verträgliche Carbonate oder Hydrogencarbonate sind zum Beispiel Alkali oder Erdalkalicarbonate, insbesondere Natriumcarbonat, Kaliumcarbonat und Calciumcarbonat, Alkali- oder Erdalkalihydrogencarbonate, insbesondere Natriumhydrogencarbonat, und Ammoniumhydrogencarbonat.

Die physiologisch verträgliche, wasserlösliche Säure oder das physiologisch verträgliche, wasserlösliche saure Salz und das physiologisch verträgliche Carbonat oder Hydrogencarbonat sind in der ersten Komponente vorzugsweise in einem äquivalenten Verhältnis vorhanden.

Der Begriff "äquivalentes" Verhältnis bedeutet hierbei, daß die molaren Einsatzmengen der Säure oder des sauren Salzes und des Carbonates oder Hydrogencarbonates so zu wählen sind, daß bei einer vollständigen Reaktion der Säure oder des sauren Salzes mit dem Carbonate oder Hydrogencarbonat eine völlige Neutralisation der Säuregruppen der Säure oder des sauren Salzes erfolgt.

Falls es jedoch aus anwendungstechnischen Gründen, zum Beispiel zur Einstellung eines bestimmten pH-Wertes des erfindungsgemäßen Haarbehandlungsmittels, erforderlich ist, können die physiologisch verträgliche, wasserlösliche Säure oder das physiologisch verträgliche, wasserlösliche saure Salz und das physiologisch verträgliche Carbonat oder Hydrogencarbonat auch in einem anderen als dem äquivalenten Verhältnis eingesetzt werden.

In der Regel besteht die erste Komponente ausschließlich aus einer physiologisch verträglichen, wasserlöslichen Säure oder einem physiologisch verträglichen, wasserlöslichen sauren Salz und einem physiologisch verträglichen Carbonat oder Hydrogencarbonat. Es ist jedoch möglich, weitere kosmetische Zusatzstoffe, wie zum Beispiel gepulverte Blätter, Blüten oder Wurzeln, pulverförmige Pflanzenextrakte, mikroverkapselte Parfüm öle oder Pflanzenöle und Pigmente oder Anfärbestoffe, dieser ersten Komponente beizumischen.

Diese Zusatzstoffe werden in den üblicherweise verwendeten Mengen eingesetzt. So werden beispielsweise die gepulverten Blätter, Blüten oder Wurzeln in einer Menge von etwa 1 bis 5 Gewichtsprozent eingesetzt, während die Einsatzmenge für die Parfümöle und Anfärbestoffe etwa 0,1 bis 1 Gewichtsprozent beträgt.

Bei der zweiten Komponente handelt es sich um ein 0,1 bis 50 Gewichtsprozent einer oberflächenaktiven Substanz enthaltendes, wasserhaltiges Haarbehandlungsmittel, das nach einer bestimmten Einwirkungszeit wieder aus dem Haar ausgespült wird und in Form einer Lösung, einer Öl-in-Wasser-Emulsion, einer Creme oder eines Gels vorliegen kann. Beispiele für ein derartiges Haarbehandlungsmittel sind Haarpflegemittel, Haarwaschmittel, Haarfärbemittel oder Haartönungsmittel.

Wenn es sich bei dem Haarbehandlungsmittel der zweiten Komponente um ein Haarpflegemittel, beispielsweise um eine Haarkur, eine Haarspülung oder einen Haarkonditioner, handelt, so liegt es vorzugsweise in Form einer Öl-in-Wasser-Emulsion vor. Es ist jedoch auch möglich, daß das Haarpflegemittel in Form einer Creme oder eines Gels vorliegt.

Als oberflächenaktive Substanzen für ein derartiges Haarpflegemittel sind Netzmittel oder Emulgatoren aus den Klassen der anionischen, kationischen, amphoteren oder nichtionischen oberflächenaktiven Substanzen, wie zum Beispiel Fettalkoholsulfate, Fettalkoholethersulfate, Alkylsulfonate, Alkylbenzolsulfate, quaternäre Ammoniumsalze, Alkylbetaine, oxethylierte Fettalkohole, oxethylierte Alkylphenole, Fettsäurealkanolamide und ox ethylierte Fettsäureester, geeignet. Als übliche Zusatzstoffe kommen ferner Verdicker, wie beispielsweise höhere Fettalkohole, Bentonit, Stärke, Polyacrylsäure, Cellulosederivate wie Carboxymethylcellulose, Hydroxypropylmethylcellulose oder kationische Celluloseether, sowie Pflegestoffe wie Lanolin und Lanolinderivate, Cholesterin, Pantothensäure und Betain, weiterhin Farbstoffe, Parfümöle, Antioxidantien, Konservierungsstoffe und Komplexbildner in Betracht. Die erwähnten oberflächenaktiven Substanzen werden in diesem Haarpflegemittel in einer Menge von 0,1 bis 30 Gewichtsprozent, vorzugsweise in einer Menge von 0,5 bis 15 Gewichtsprozent, eingesetzt, während die Zusatzstoffe in den für solche Zwecke üblichen Mengen verwendet werden. So können zum Beispiel die Verdicker in einer Menge von etwa 0,1 bis 25 Gewichtsprozent in den Zubereitungen enthalten sein.

Für die Verwendung als oberflächenaktive Substanzen sind hierbei quaternäre Ammoniumsalze, beispielsweise Oxyethylalkylammoniumphosphate, Alkyltrimethylammoniumchloride, Dialkyldimethylammoniumchloride, Alkyldimethylbenzylammoniumchloride oder Alkylpyridiniumchloride, besonders gut geeignet.

Liegt das Haarpflegemittel in Form einer Öl-in-Wasser-Emulsion oder einer Creme vor, so enthält es zusätzlich zu den vorstehend genannten oberflächenaktiven Substanzen und Zusatzstoffen eine Öl- oder Wachskomponente. Als geeignete Öle und Wachse können beispielsweise genannt werden: natürliche Öle und Wachse wie zum Beispiel Süßmandelöl, Avocadoöl, Olivenöl und Bienenwachs, Fettalkohole, Fettsäureester, Fettsäureamide, Vaseline, Wollfettalkohol, Wollfett und feste oder flüssige Paraffine.

Handelt es sich bei dem Haarbehandlungsmittel der zweiten Komponente um eine Haarfärbemittel oder Haartönungsmittel, so liegt es vorzugsweise in Form einer Öl-in-Wasser-Emulsion, einer Creme oder eines Geles vor und enthält dieselben oberflächenaktiven Substanzen und Zusatzstoffe wie die vorstehend genannten Haarpflegemittel.

Zusätzlich zu diesen Stoffen enthalten diese Haarfärbemittel und Haartönungsmittel jedoch einen oder mehrere für die Haarfärbung geeignete Verbindungen aus der Gruppe der direkt auf das Haar aufziehenden Farbstoffe wie zum Beispiel aromatische Nitrofarbstoffe, Azofarbstoffe, Anthrachinonfarbstoffe und Triphenylmethanfarbstoffe, oder Oxidationsfarbstoffe wie zum Beispiel aromatische Diamine und Aminophenole, wobei der Farbstoffgehalt dieser Mittel üblicherweise etwa 0,1 bis 5 Gewichtsprozent beträgt.

Derartige für die Haarfärbung geeignete Farbstoffe werden unter anderem in dem Buch von J. C.

Johnson "Hair Dyes", Noyes Data Corp., Park-Ridge / USA (1973), auf den Seiten 3 bis 91 und 113 bis 139 (ISBN: 0-8155-0477-2), in dem Buch von E. Sagarin "Cosmetics, Science and Technology", Interscience Publishers Inc., New York (1957), auf den Seiten 503 ff. und in dem Buch von H. Janistyn, "Handbuch der Kosmetika und Riechstoffe", Band 3, Dr. Alfred Hüthig Verlag, Heidelberg (1973) auf den Seiten 338 ff., beschrieben.

Handelt es sich bei dem Haarbehandlungsmittel der zweiten Komponente um ein Haarwaschmittel, so liegt es vorzugsweise in Form einer wäßrigen Lösung vor. Es ist jedoch ebenfalls möglich, als Zubereitungsform eine Öl-in-Wasser-Emulsion, eine Creme oder ein Gel zu wählen.

Dieses Haarwaschmittel enthält im allgemeinen 3 bis 50 Gewichtsprozent, vorzugsweise 3 bis 20 Gewichtsprozent, der oberflächenaktiven Substanz und weist einen pH-Wert zwischen 3 und 9, vorzugsweise zwischen 4 und 7, auf.

Weiterhin kann dieses Haarwaschmittel weitere übliche Zusatzstoffe, insbesondere Parfümöle, Konservierungsstoffe, Verdicker, Schaumstabilisatoren, Puffersubstanzen, kosmetische Polymere, Pigmente und Farbstoffe enthalten.

Unter den Schaumstabilisatoren können die Fettamide und insbesondere die Mono- oder Diethanolamide von Kokosfettsäuren, Lauryl- oder Ölsäuremono- oder -diethanolamid, die zweckmäßigerweise in Mengen von 1 bis 10 und vorzugsweise von 1 bis 3 Gewichtsprozent Verwendung finden, angeführt werden.

Unter den Verdickern können insbesondere Acrylpolymere und Cellulosederivate, wie zum Beispiel kationische Celluloseether, Carboxymethylcellulose, Hydroxypropylmethylcellulose und Hydroxyethylcellulose, angeführt werden. Die Verdicker liegen im allgemeinen in einem Anteil von 0,1 bis 5 Gewichtsprozent vor.

Unter den oberflächenaktiven Substanzen, die für die Verwendung in diesem Haarwaschmittel geeignet sind, sind beispielsweise die folgenden zu nennen:

a) die anionischen oberflächenaktiven Substanzen, wie beispielsweise die Alkali-, Erdalkali-, Ammonium- oder Alkanolaminsalze von Alkansulfonaten, Alkylsulfaten, Alkylarylsulfaten und Alkylethersulfaten, die $C_{12}$ bis $C_{18}$-Alkyl- und insbesondere $C_{12}$ bis $C_{14}$-Alkyl-Sulfatnatriumsalze oder -Triethanolaminsalze, die Natrium oder Triethanolaminsalze von Lauryl- oder Tetradecylethersulfaten, das Dinatriumsalz des Sulfosuccinhalbesters von Alkanolamiden, die Seifen, die Polyethercarbonsäuren und die Fettsäuremonoglyceridsulfate;

b) die nichtionischen oberflächenaktiven Substanzen, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycolether von gesättigten oder ungesättigten Fettalkoholen, Fettsäuren und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Substanzen, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder - bromid, die Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, die Alkylamidethyltrimethylammoniumethersulfate, die Alkyldimethylammoniumsaccharinate, die Pentaoxyethylammoniumchloride, die Alkyldimethyldichlorbenzylammoniumchloride, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Substanzen, wie beispielsweise die Carboxylderivate von Imidazol, die N-Alkylbetaine, die N-Alkylamidobetaine, die N-Alkylsulfobetaine, die N-Alkylaminoproprionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearat-betain.

Das erfindungsgemäße schaumförmige Haarbehandlungsmittel wird vorzugsweise durch Vermischen eines Gewichtsteiles der vorstehend beschriebenen ersten Komponente mit 3 bis 11 Gewichtsteilen der vorstehend beschriebenen zweiten Komponente erhalten. Dieses schaumförmige Haarbehandlungsmittel besitzt einen voluminösen, feinporigen und sehr stabilen Schaum dessen Schaumeigenschaften denen von Aerosolschaumpräparaten gleichwertig sind. Das erfindungsgemäße schaumförmige Haarbehandlungsmittel besitzt gegenüber Aerosolschaumpräparaten den Vorteil, daß es frei von umweltgefährdenden und brennbaren Treibgasen ist und kostengünstiger hergestellt werden kann.

Ein weiterer Gegenstand der vorliegenden Anmeldung ist ein Verfahren zur Herstellung des erfindungsgemäßen schaumförmigen Haarbehandlungsmittels, bei dem man vor dem Gebrauch des Haarbehandlungsmittels ein (a) eine physiologisch verträgliche, wasserlösliche Säure oder ein physiologisch verträgliches, wasserlösliches saures Salz sowie (b) ein physiologisch verträgliches, Carbonat oder Hydrogencarbonat enthaltendes Pulver oder Granulat (Komponente 1) mit einem emulsionsförmigen, gelförmigen, cremeförmigen oder in Form einer Lösung vorliegenden wasserhaltigen Haarbehandlungsmittel, welches 0,1 bis 50 Gewichtsprozent einer oberflächenaktiven Substanz enthält, (Komponente 2) vermischt.

Zur Herstellung des erfindungsgemäßen Mittels werden eine ausreichende Menge der Komponente 1 und der Komponente 2, vorzugsweise in einer Schale oder einem Becherglas, mit einem Pinsel oder einem Spatel etwa 15 bis 60 Sekunden lang, vorzugsweise 20 bis 30 Sekunden lang, miteinander vermischt.

Die Komponente 1 und die Komponente 2 werden hierbei, in Abhängigkeit von der Viskosität der Komponente 2, vorzugsweise in einem Gewichtsverhältnis von 1 : 3 bis 1 : 11 miteinander vermischt. In der Regel ist bei Verwendung einer hochviskosen Komponente 2 eine größere Menge der Komponente 1 erforderlich als bei Verwendung der gleichen Menge einer niedrigviskosen Komponente 2.

Prinzipiell ist es auch möglich, daß erfindungsgemäße schaumförmige Haarbehandlungsmittel durch Vermischen zweier Komponenten herzustellen, deren erste Komponente entweder (a) eine physiologisch verträgliche, wasserlösliche Säure beziehungsweise ein physiologisch verträgliches, wasserlösliches saures Salz oder aber (b) ein physiologisch verträgliches Carbonat beziehungsweise Hydrogencarbonat enthält, während die jeweils andere der beiden vorgenannten Verbindungen in dem als zweite Komponente verwendeten wasserhaltigen Haarbehandlungsmittel enthalten ist, wobei jedoch zu beachten ist, daß das Mischungsverhältnis der beiden Komponenten so gewählt wird, daß die Verbindungen (a) und (b) in einem äquivalenten Verhältnis eingesetzt werden.

Eine derartige Verfahrensweise besitzt jedoch einige anwendungstechnische Nachteile:

So müssen zum Beispiel die erste und die zweite Komponente sehr genau abgewogen werden und anschließend in einem vorher genau berechneten Mengenverhältnis miteinander vermischt werden, da ansonsten der für das schaumförmige Haarbehandlungsmittel erforderliche pH-Wert verändert wird. Ebenfalls kann der pH-Wert der zweiten Komponente durch den Zusatz der Säure beziehungsweise des sauren Salzes oder des Carbonates beziehungsweise Hydrogencarbonates derart verändert werden, daß die Lagerstabilität dieser Komponente erheblich beeinträchtigt wird.

Aus diesem Grunde ist das zuerst genannte Herstellungsverfahren vorzuziehen.

Die nachfolgenden Beispiele sollen den Gegenstand der Erfindung näher erläutern:

## Beispiele

| Beispiel 1: Haarspülung | | |
|---|---|---|
| Komponente 1: | 43,3 g<br>56,7 g<br>$\overline{100,0\ g}$ | Zitronensäure, wasserfrei<br>Natriumhydrogencarbonat, wasserfrei |
| Komponente 2: (emulsionsförmig) | 3,0 g<br>1,0 g<br>1,0 g<br>1,0 g<br>94,0 g<br>$\overline{100,0\ g}$ | Stearylalkohol<br>Lanolin (Adeps lanae)<br>Vaseline<br>Tris-(oligooxyethyl)-octadecylammoniumphosphat<br>Wasser vollentsalzt |

30 g der Komponente 2 werden in einer Schale mit 5 g der Komponente 1 vermischt (Dauer: 20 Sekunden). Sodann wird der erhaltene voluminöse, feinporige Schaum auf dem Haar gleichmäßig verteilt. Nach einer Einwirkungszeit von 3 Minuten wird das Haar mit Wasser gründlich ausgespült.

| Beispiel 2: Haarkonditioner | | |
|---|---|---|
| Komponente 1: | 58,6 g<br>41,4 g<br>100,0 g | Weinsäure, wasserfrei<br>Natriumcarbonat, wasserfrei |
| Komponente 2:<br>(emulsionsförmig) | 4,0 g<br>2,5 g<br>1,0 g<br>92,5 g<br>100,0 g | Cetylstearylalkohol<br>Kokospentaethoxymethylammoniumchlorid<br>Sorbitanmonopalmitat, oxethyliert mit 20 Mol Ethylenoxid<br>Wasser, vollentsalzt |

Die Anwendung erfolgt in der in Beispiel 1 beschriebenen Weise.

| Beispiel 3: Haarspülung | | |
|---|---|---|
| Komponente 1: | 60,0 g<br>39,9 g<br>0,1 g<br>100,0 g | Weinsäure, wasserfrei<br>Calciumcarbonat, wasserfrei<br>Farbstoff |
| Komponente 2:<br>(emulsionsförmig) | 5,0 g<br>1,1 g<br>0,4 g<br>93,5 g<br>100.0 g | Cetylalkohol<br>Lauryldimethylbenzylammoniumchlorid<br>Dimethyl-carboxymethylenpropylenamido-stearat-betain<br>Wasser vollentsalzt |

Die Anwendung erfolgt in der in Beispiel 1 beschriebenen Weise.

| Beispiel 4: Haarkur | | |
|---|---|---|
| Komponente 1: | 42,9 g<br>57,1 g<br>100,0 g | Malonsäure, wasserfrei<br>Kaliumcarbonat, wasserfrei |
| Komponente 2:<br>(gelförmig) | 1,0 g<br>0,5 g<br>0,5 g<br>98,0 g<br>100,0 g | kationischer Celluloseether (Polymer ® JR 30 M der Union Carbide Co., USA)<br>Cetyltrimethylammoniumchlorid<br>Laurylpyridiniumchlorid<br>Wasser, vollentsalzt |

30 g der Komponente 2 werden in einer Schale mit Hilfe eines Pinsels mit 6 g der Komponente 1 vermischt (Dauer: 30 Sekunden). Der erhaltene Schaum wird sodann auf das Haar aufgetragen. Nach einer Einwirkungszeit von 3 Minuten wird das Haar mit lauwarmem Wasser ausgespült.

| Beispiel 5: Haarpflegemittel | | |
|---|---|---|
| Komponente 1: | 44,4 g<br>55,6 g<br>100,0 g | Äpfelsäure, wasserfrei<br>Natriumhydrogencarbonat, wasserfrei |
| Komponente 2: (emulsionsförmig) | 4,00 g<br>1,50 g<br>1,25 g<br>0,10 g<br>93,15 g<br>100,00 g | Cetylalkohol<br>Vaseline<br>Tris-(oligooxyethyl)-octadecylammoniumphosphat<br>Farbstoff<br>Wasser, vollentsalzt |

Die Anwendung erfolgt in der in Beispiel 1 beschriebenen Weise.

| Beispiel 6: Haarwaschmittel | | |
|---|---|---|
| Komponente 1: | 44,7 g<br>50,7 g<br>4,6 g<br>100,0 g | Glyoxylsäure<br>Natriumhydrogencarbonat, wasserfrei<br>getrocknete und gepulverte Kamillenblüten |
| Komponente 2: | 15,0 g<br>85,0 g<br>100,0 g | Dimethyl-carboxymethylenpropylenamido-stearat-betain<br>Wasser, vollentsalzt |

30 g der Komponente 2 werden mit 4 g der Komponente 1 vermischt (Dauer: 20 Sekunden). Anschließend wird die Hälfte des erhaltenen Schaumes auf dem Haar verteilt und sodann in die Haare einmassiert. Nach 1 Minute wird das Haar mit Wasser gespült und in der beschriebenen Weise mit der restlichen Schaummenge nochmals behandelt. Anschließend spült man das Haar mit Wasser.

| Beispiel 7: Anionisches Haarwaschmittel | | |
|---|---|---|
| Komponente 1: | 67,2 g<br>32,8 g<br>100,0 g | Natriumhydrogentartrat, wasserfrei<br>Natriumhydrogencarbonat, wasserfrei |
| Komponente 2: | 11,200 g<br>4,000 g<br>0,050 g<br>0,025 g<br>84,725 g<br>100,000 g | Laurylalkoholdiglykolethersulfat-Natriumsalz<br>Natriumchlorid<br>Farbstoff<br>Formaldehyd<br>Wasser, vollentsalzt |

Die Anwendung erfolgt in der in Beispiel 6 beschriebenen Weise.

| Beispiel 8: Amphoteres, tönendes Haarwaschmittel | | |
|---|---|---|
| Komponente 1: | 42,9 g<br>57,1 g<br>$\overline{100,0\text{ g}}$ | Malonsäure, wasserfrei<br>Kaliumcarbonat, wasserfrei |
| Komponente 2: | 14,00 g<br>3,50 g<br>0,51 g<br>0,01 g<br>81,98 g<br>$\overline{100,00\text{ g}}$ | Dimethyl-carboxymethylenpropylenamido-stearat-betain<br>Kokosfettsäurediethanolamid<br>Ameisensäure<br>Pikraminsäure<br>Wasser, vollentsalzt |

Die Anwendung erfolgt in der in Beispiel 6 beschriebenen Weise.

| Beispiel 9: Haartönungsmittel | | |
|---|---|---|
| Komponente 1: | 44,4 g<br>55,6 g<br>$\overline{100,0\text{ g}}$ | Äpfelsäure, wasserfrei<br>Natriumhydrogencarbonat, wasserfrei |
| Komponente 2: (emulsionsförmig) | 12,00 g<br>1,68 g<br>0,85 g<br>0,50 g<br>0,50 g<br>0,10 g<br>84,37 g<br>$\overline{100,00\text{ g}}$ | Cetylstearylalkohol<br>Laurylalkohol-diglykolethersulfat-Natriumsalz<br>1,4-Diamino-2-nitrobenzol<br>1-Hydroxy-2-amino-4-nitrobenzol<br>Parfümöl<br>Parahydroxybenzoesäureethylester<br>Wasser, vollentsalzt |

30 g der Komponente 2 werden in einer Schale mit Hilfe eines Pinsels 20 Sekunden lang mit 5 g der Komponente 1 vermischt. Der erhaltene Schaum wird sodann mit dem Pinsel auf das Haar aufgetragen. Nach einer Einwirkungszeit von 15 Minuten wird das Haar mit Wasser gespült.

| Beispiel 10: Oxidationshaarfärbemittel | | |
|---|---|---|
| Komponente 1: | 43,3 g<br>56,7 g<br>$\overline{100,0\text{ g}}$ | Zitronensäure, wasserfrei<br>Natriumhydrogencarbonat, wasserfrei |
| Komponente 2a: | 15,00 g<br>0,98 g<br>0,30 g<br>0,30 g<br>0,25 g<br>0,08 g<br>83,09 g<br>$\overline{100,00\text{ g}}$ | Cetylalkohol<br>Laurylalkohol-diglykolethersulfat-Natriumsalz<br>Natriumsulfit<br>1,4-Diaminobenzol<br>Resorcin<br>3,5-Diamino-2,6-dimethoxypyridindihydrochlorid<br>Wasser, vollentsalzt |
| Komponente 2b: | 50 ml Wasserstoffperoxid (12-prozentige wäßrige Lösung) | |

50 g der Komponente 2a werden mit 25 ml der Komponente 2b vermischt. Anschließend wird diese Mischung (= Komponente 2) 25 Sekunden lang mit 7 g der Komponente 1 verrührt. Der so erhaltene feinporige Schaum wird auf das Haar aufgetragen und nach einer Einwirkungszeit von 30 Minuten mit lauwarmem Wasser wieder ausgespült.

Alle Prozentangaben betreffen, sofern nicht anders angegeben, Gewichtsprozente.

EP 0 399 157 A2

**Ansprüche**

1. Schaumförmiges Haarbehandlungsmittel, welches vor der Anwendung durch Vermischen zweier Komponenten hergestellt wird, dadurch gekennzeichnet, daß (1) die erste Komponente in fester Form, als Pulver oder Granulat, vorliegt und eine physiologisch verträgliche, wasserlösliche Säure oder ein physiologisch verträgliches, wasserlösliches saures Salz sowie ein physiologisch verträgliches, Carbonat oder Hydrogencarbonat enthält und (2) die zweite Komponente ein wasserhaltiges Haarbehandlungsmittel ist und 0,1 bis 50 Gewichtsprozent einer oberflächenaktiven Substanz enthält.

2. Mittel nach Anspruch 1, dadurch gekennzeichnet, daß die physiologisch verträgliche, wasserlösliche Säure und das physiologisch verträgliche, wasserlösliche saure Salz ausgewählt sind aus Zitronensäure, Äpfelsäure, Weinsäure, Ascorbinsäure, Mandelsäure, Glyoxylsäure, Malonsäure, Natriumhydrogentartrat und Natriumhydrogensulfat.

3. Mittel nach Anspruch 1 oder 2, dadurch gekennzeichnet, daß das physiologisch verträgliche, Carbonat oder Hydrogencarbonat ausgewählt ist aus Alkali- oder Erdalkalicarbonaten, Alkali- oder Erdalkalihydrogencarbonaten und Ammoniumhydrogencarbonat.

4. Mittel nach einem der Ansprüche 1 bis 3, dadurch gekennzeichnet, daß es in Form einer Lösung, einer Öl-in-Wasser-Emulsion, einer Creme oder eines Gels vorliegt.

5. Mittel nach einem der Ansprüche 1 bis 4, dadurch gekennzeichnet, daß die zweite Komponente ein Haarpflegemittel, ein Haarwaschmittel, ein Haarfärbemittel oder ein Haartönungsmittel ist.

6. Mittel nach einem der Ansprüche 1 bis 5, dadurch gekennzeichnet, daß die physiologisch verträgliche, wasserlösliche Säure oder das physiologisch verträgliche, wasserlösliche saure Salz und des physiologisch verträgliche Carbonat oder Hydrogencarbonat in der ersten Komponente in einem äquivalenten Verhältnis enthalten sind.

7. Mittel nach einem der Ansprüche 1 bis 6, dadurch gekennzeichnet, daß es durch Vermischen von 1 Gewichtsteil der ersten Komponente mit 3 bis 11 Gewichtsteilen der zweiten Komponente erhalten wird.

8. Verfahren zur Herstellung eines schaumförmigen Haarbehandlungsmittels, dadurch gekennzeichnet, daß man vor dem Gebrauch des Haarbehandlungsmittels ein (a) eine physiologisch veträgliche, wasserlösliche Säure oder ein physiologisch verträgliches, wasserlösliches saures Salz sowie (b) ein physiologisch verträgliches Carbonat oder Hydrogencarbonat enthaltendes Pulver oder Granulat (Komponente 1) mit einem emulsionsförmigen, gelförmigen, cremeförmigen oder in Form einer Lösung vorliegenden, wasserhaltigen Haarbehandlungsmittel, welches 0,1 bis 50 Gewichtsprozent einer oberflächenaktiven Substanz enthält, (Komponente 2) vermischt.

9. Verfahren nach Anspruch 8, dadurch gekennzeichnet, daß die Komponente 1 und die Komponente 2 in einem Gewichtsverhältnis von 1 : 3 bis 1 : 11 miteinander vermischt werden.

10. Verfahren nach Anspruch 8 oder 9, dadurch gekennzeichnet, daß man die Komponente 1 zur Komponente 2 gibt und die beiden Komponenten anschließend mit einem Pinsel oder Spatel 15 bis 60 Sekunden lang miteinander vermischt.

9